# EUROPEAN PATENT SPECIFICATION

(11) **EP 4 185 874 B1**
(45) Date of publication and mention of the grant of the patent: **06.11.2024**
(21) Application number: 21759376.3
(22) Date of filing: 21.07.2021
(51) Int. Cl.: G01N 33/68

(54) **A DIAGNOSTIC BLOOD TEST METHOD FOR THE DETECTION OF ALZHEIMER'S DISEASE**
DIAGNOSTISCHES BLUTTESTVERFAHREN ZUM NACHWEIS VON ALZHEIMER
MÉTHODE D'ANALYSE SANGUINE DE DIAGNOSTIC POUR LA DÉTECTION DE LA MALADIE D'ALZHEIMER

(30) Priority: 22.07.2020 HU 2000240
(43) Date of publication of application: 31.05.2023
(73) Proprietor: Pharmacoidea Kft, 6726 Szeged (HU)
(72) Inventor: HUDÁK, Anett, Damjanich utca 110/1 (HU); LETOHA, Tamás, Liliom utca 12 (HU)
(74) Representative: Marmarosi, Tamasné
(86) International application number: PCT/HU2021/000011
(87) International publication number: WO 2022/018468

(56) References cited:
- WO-A1-2020/115513
- ZHANG GAN-LIN ET AL: "Towards Understanding the Roles of Heparan Sulfate Proteoglycans in Alzheimer's Disease", BIOMED RESEARCH INTERNATIONAL, vol. 2014, 1 January 2014 (2014-01-01), pages 1 - 9, XP055868834, ISSN: 2314-6133, DOI: 10.1155/2014/516028
- SMITH PATRICE D ET AL: ""GAG-ing with the neuron": The role of glycosaminoglycan patterning in the central nervous system", EXPERIMENTAL NEUROLOGY, ELSEVIER, AMSTERDAM, NL, vol. 274, 12 August 2015 (2015-08-12), pages 100 - 114, XP029329847, ISSN: 0014-4886, DOI: 10.1016/J.EXPNEUROL.2015.08.004
- BONNEH-BARKAY D ET AL: "Brain extracellular matrix in neurodegeneration", BRAIN PATHOLOGY, ZUERICH, CH, vol. 19, no. 4, 25 July 2008 (2008-07-25), pages 573 - 585, XP002591297, ISSN: 1015-6305, DOI: 10.1111/J.1750-3639.2008.00195.X
- TIAN LI ET AL: "Decreased Expression of Cathepsin D in Monocytes is Related to the Defective Degradation of Amyloid-[beta] in Alzheimer's Disease", JOURNAL OF ALZHEIMER`S DISEASE, vol. 42, no. 2, 28 August 2014 (2014-08-28), NL, pages 511 - 520, XP055870353, ISSN: 1387-2877, Retrieved from the Internet <URL:http://dx.doi.org/10.3233/JAD-132192> DOI: 10.3233/JAD-132192

## Description

The present invention relates to a predictive diagnostic method for detecting Alzheimer's disease, by measuring the level of syndecan-3 expressed by circulating monocytes in the blood.

Monocytes are important cell types of the innate immune system playing key roles in the protection against invasive pathogens, clearing dead cells and cell debris by phagocytosis [Fiala et al. 2005 (J. Alzheimer's Dis. 7 (3): 255-262)]. Monocytes are also involved in the pathogenesis of many diseases. In Alzheimer's disease, which is associated with abnormal CNS degeneration, infiltrating monocytes play an important role in eliminating circulating amyloid-beta (Aβ) microaggregates and brain Aβ plaques. In chronic inflammatory or autoimmune diseases, the frequency of monocytes in the blood increases.

White blood cells involved in the inflammatory response, such as monocytes, express several cellsurface molecules. Due to their highly diverse heparan sulfate (HS) side chains, heparan sulfate proteoglycans (HSPGs), one of the most populous families of cell surface membrane proteins, play an important role as receptors in the signaling processes of many cytokines and growth factors [Williams and Fuki 1997 (Curr. Opin. Lipidol 8 (5), 253-262), Bishop, Schuksz et al. 2007 (Nature 446 (7139) 1030-1037), Couchman 2010 (Annu. Rev. Cell Dev. Biol. 26, 89-114), lozzo and Karamanos 2010 (FEBS J. 277 (19), 3863)]. Two types of HSPGs are found in mammalian cells: CNS-expressed glypicans that are anchored to the membrane via glycosylphosphatidylinositol and the ubiquitous transmembrane syndecans [Christianson and Belting 2014 (Matrix Biol. 35, 51-55)]. Four isoforms of syndecans are known: syndecan-1 expressed by epithelial and plasma cells, syndecan-2 by endothelial cells and fibroblasts, syndecan-3 by neurons, and the universally expressed syndecan-4 [Tkachenko, Rhodes et al. 2005 (Circ. Res. 96 (5), 488-500), Afratis, Nikitovic et al. 2017 (FEBS. J. 284 (1), 27-41)].

Syndecan-3 (N-syndecan or neuronal syndecan) is a 442 amino acid long transmembrane protein mainly expressed by neurons [Tkachenko, Rhodes et al. 2005 (Ther. Deliv. 4 (12), 1479-1481)]. Syndecan-3 has four conserved glycosaminoglycan binding sites at the N-terminus, along with unique threonine-rich and mucin-like regions close to the membrane. The heparan sulfate chains of syndecan-3 serve as binding sites for several growth factors, including AgRP (Agouti-related protein), HB-GAM (heparin-binding growth-associated molecule), GDNF (glial cell line-derived growth factor), NRTN (neurturin), artemin and NOTCH [Bespalov, Sidorova et al. 2011 (J Cell Biol. 192 (1): 153-169), Creemers, Pritchard et al. 2006 (Endocrinology. 147 (4): 1621-1631), Nolo, Kaksonen et al. 1995 (Neurosci Lett. 191 (1-2): 39-42), Pisconti, Cornelison et al. 2010 (J Cell Biol. 190 (3): 427-441), Reizes, Benoit et al. 2003 (Ann N Y Acad Sci. 994: 66-73)]. Syndecan-3 also plays a role in regulating memory and body weight/metabolism [Kaksonen, Pavlov et al. 2002 (Mol. Cell. Neurosci. 21 (1), 158-172), Strader, Reizes et al. 2004 (J. Clin. Invest. 114 (9 (, 1354-1360), Reizes, Benoit et al. 2008 (Int. J. Bioche. Cell. Biol. 40 (1), 28-45)]. The expression of syndecan-3, along with syndecan-4, is elevated in the brains of Alzheimer's patients [Liu, Zhao et al. 2016 (Sci. Trans. Med. 8 (332), 332-344)].

In addition to its role in the CNS, little is known about the role of syndecan-3 in the periphery. However, we know that syndecan-3 plays an important role in viral infections: syndecan-3, as an HIV-1 receptor, mediates HIV-1's transmission to T cells [de Witte, Bobardt et al. 2007 (Nat. Acad. Sci. USA 10449, 19464-19469)]. It is also known that syndecan-3 is expressed by circulating monocytes [Wegrowski, Milard et al. 2006 (Clin Exp Immunol. Jun; 144 (3): 485-493)]. Syndecan-3 present on monocytes is a good indicator of the inflammatory processes [Jackson 1997 (Biochem Soc Trans. 25 (1): 220-49)].

We aimed to investigate syndecan-3 expressed on monocytes as a prognostic biomarker in neurodegenerative diseases such as Alzheimer's.

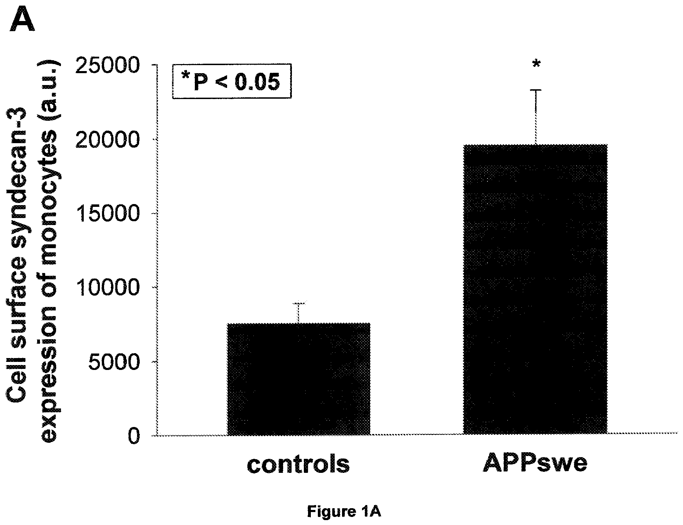

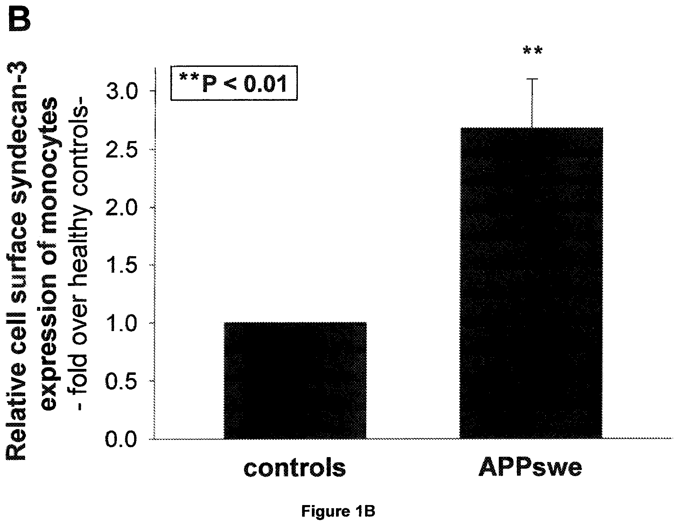

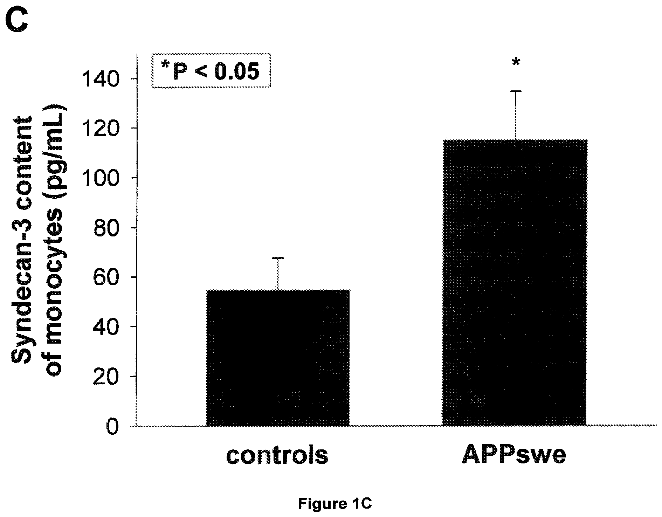

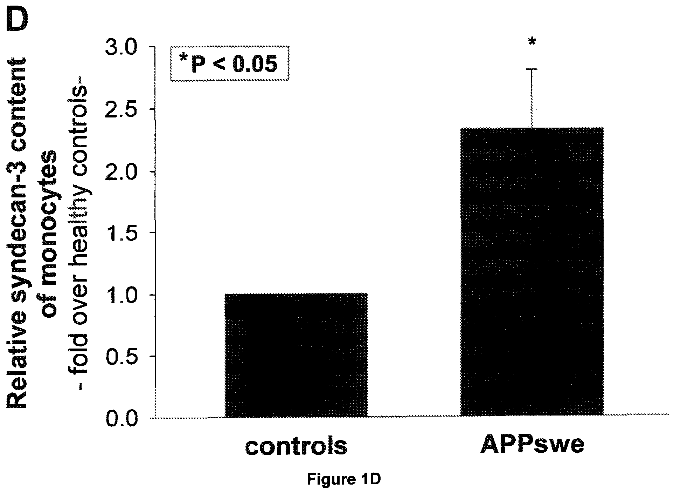

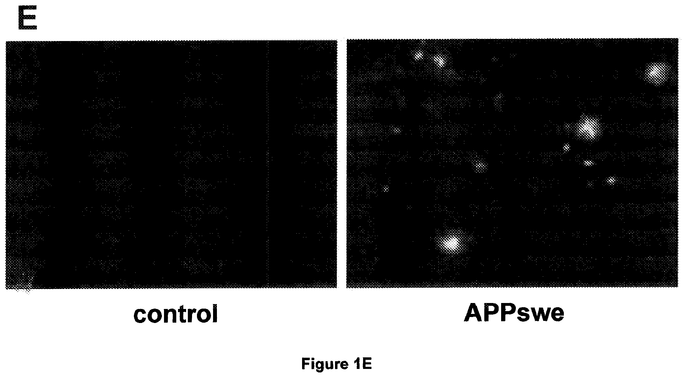

Our experiments examined changes in syndecan-3 levels in circulating monocytes in an APPSWE-Tau mouse model of Alzheimer's disease. Syndecan-3 levels of circulating monocytes were examined in blood samples from APPSWE-Tau (Taconic Biosciences) and healthy C57BL/6 mice with a minimum age of 6 months. The number of animals in the healthy control and APPSWE-Tau groups was 10-10, with an equal number of males (5-5 individuals) and females (5-5 individuals) in both groups. Blood of mice anesthetized with Avertin was collected with cardiac puncture. After transcardinal perfusion with ice-cold PBS (2 mL/min, 8 min), the brain was removed, cut in half frontally, and frozen in dry ice for further microscopic examinations. Bloodderived monocytes were isolated with the EasySep^{™} Mouse Monocyte Isolation Kit (Stemcell Technologies, cat. no. 19861), while brain samples were stained for Alzheimer's disease plaques to check the severity of the brain lesion in the animals. Assessment of cell surface syndecan-3 expression and syndecan-3 content of monocytes showed that both cell surface syndecan-3 expression and syndecan-3 content of monocytes isolated from diseased mice were significantly (p<0.05) higher than those of healthy controls (Figs. 1A-D). (Cell surface syndecan-3 expression of monocytes were measured with an AMNIS FlowSight flow cytometer using APC-labeled human syndecan-3 antibody [R&D Systems, Catalog No. FAB3539A], while syndecan-3 content of whole monocyte lysates was measured with a mouse syndecan-3 ELISA kit (PicoKine^{™}, Boster Biological Technology, Pleasanton CA, USA, cat. no. EK1556; absorbance was measured with a BioTek Cytation 3 Cell Imaging Multi-Mode Reader). For each flow cytometric measurement, the expression of 10,000 monocytes of syndecan-3 was examined. After perfusion, the brains were isolated, frontally cut and fixed in 4% paraformaldehyde for 18 h. Then 30 µm thick sagittal sections were cut and Aβ plaques were stained with Aβ specific antibody (MOAB-2, NOVUS Biologicals, cat. number: NBP2-13075) and 488-labeled secondary antibody (ThermoFischer Scientific, cat. no. A-21141) The number and size of Aβ plaques deposition in the resulting brain sections were assessed with a BioTek Cytation 3 Cell Imaging Multi-Mode Reader (Figs. 1E).

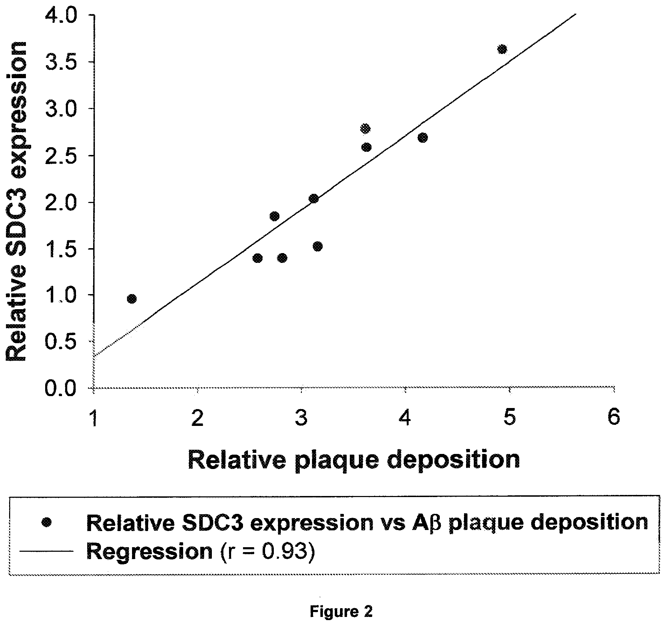

Syndecan-3 levels of monocytes isolated from the blood correlated with the extents of plaque deposition in the brains of APPSWE-Tau mice: i.e., Aβ plaque deposition in the central nervous system was associated with high syndecan-3 expression by blood monocytes (the value of Pearson's correlation coefficient [r] between CNS plaque deposition and syndecan-3 expression of monocyte is 0.93 - see Figure 2.)

The present invention is based on the surprising finding that the level of syndecan-3 in circulating monocytes in Alzheimer's disease is significantly increased, such that the level of syndecan-3 in monocytes isolated from blood correlates with CNS plaque deposition.

The present invention relates to a method for diagnosing Alzheimer's disease, by measuring the syndecan-3 expression of monocytes isolated from the systemic circulation with an antibody or any other ligand specific to the 442 amino acid protein core of syndecan-3 and the deviation from the healthy controls is evaluated.

Specific antibodies or ligands are known to those skilled in the art, for example, from the following literature Hudák, Kusz, Domonkos et al. 2019 (Sci Rep 9, 16543).

The specificity of the method is ensured by the specific binding of the antibody or ligand to any part of the 442 amino acid long core protein of syndecan-3.

Syndecan-3-specific antibody or ligand alone or attached to a surface binds to syndecan-3 expressed on monocytes, so it can be used as a diagnostic method in neurodegenerative diseases.

The syndecan-3 specific antibody or ligand can be contacted with blood monocytes alone or in conjunction with a fluorescent or other optical signaling agent.

As a control value, the following mean value (± SEM) were measured with a BioTek Cytation 3 Cell Imaging Multi-Mode Reader: 54.70 pg (± 12.96) syndecan-3 / mL, while measuring the syndecan-3 expression of monocytes isolated from healthy controls with Amnis^{®} FlowSight^{®} Imaging Flow Cytometer, the following fluorescence mean value (± SEM) was obtained: 7546.71 ± 1329.47 arbitary unit (a.u.) / cell

The data is for information only and depends on the measurement techniques, so the scope of the invention is not limited to the arbitary values given here.

## Claims

1. Method for diagnosing Alzheimer's disease, **characterized by** measuring the syndecan-3 expression of monocytes isolated from the systemic circulation with an antibody or any other ligand specific to the 442 amino acid protein core of syndecan-3 and evaluating the deviation from the healthy controls.

2. The method of claim 1, **characterized by**, that the antibody or ligand specific to any portion of the 442 amino acid long protein core of syndecan-3, is present in soluble form or bound to a surface.

## Patentansprüche

1. Verfahren zur Diagnose der Alzheimer-Krankheit, **gekennzeichnet durch** Messung der Syndecan-3-Expression von Monozyten, die aus dem systemischen Kreislauf isoliert wurden, mit einem Antikörper oder einem anderen Liganden, der spezifisch für den 442-Aminosäure-Proteinkern von Syndecan-3 ist, und Bewertung der Abweichung von den gesunden Kontrollen.

2. Verfahren nach Anspruch 1, **dadurch gekennzeichnet, dass** der Antikörper oder Ligand, der spezifisch für einen beliebigen Teil des 442 Aminosäuren langen Proteinkerns von Syndecan-3 ist, in löslicher Form vorliegt oder an die Oberfläche gebunden ist.

## Revendications

1. Méthode de diagnostic de la maladie d'Alzheimer, **caractérisée par** la mesure de l'expression de syndécane-3 des monocytes isolés de la circulation systémique à l'aide d'un anticorps ou de tout autre ligand spécifique au noyau protéique de 442 acides aminés du syndécane-3, et par l'évaluation de l'écart par rapport aux témoins sains.

2. Méthode selon la revendication 1, **caractérisée en ce que** l'anticorps ou le ligand spécifique à une partie quelconque du noyau protéique long de 442 acides aminés du syndécane-3, est présent sous forme soluble ou lié à une surface.
